(19) Europäisches Patentamt
European Patent Office
Office européen des brevets

(11) **EP 3 994 446 B1**

(12) **EUROPEAN PATENT SPECIFICATION**

(45) Date of publication and mention
of the grant of the patent:
**31.12.2025 Bulletin 2026/01**

(21) Application number: **20834933.2**

(22) Date of filing: **06.07.2020**

(51) International Patent Classification (IPC):
*G01N 27/327* (2006.01)     *G01N 31/22* (2006.01)
*C09K 9/02* (2006.01)       *C12Q 1/6825* (2018.01)
*H01M 4/86* (2006.01)       *G01N 21/78* (2006.01)
*G01N 33/543* (2006.01)

(52) Cooperative Patent Classification (CPC):
(C-Sets available)
**G01N 31/22; C09K 9/02; C12Q 1/6825;
G01N 27/3276; H01M 4/8631;** G01N 21/78;
G01N 33/5438                          (Cont.)

(86) International application number:
**PCT/US2020/040902**

(87) International publication number:
**WO 2021/003480 (07.01.2021 Gazette 2021/01)**

(54) **ELECTRODES EMPLOYING APATAMER-BASED RECOGNITION FOR COLORIMETRIC VISUALIZATION**

ELEKTRODEN MIT APATAMERBASIERTER ERKENNUNG ZUR KOLORIMETRISCHEN VISUALISIERUNG

ÉLECTRODES UTILISANT UNE RECONNAISSANCE À BASE D'APTAMÈRES POUR VISUALISATION COLORIMÉTRIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.07.2019 US 201962870392 P**

(43) Date of publication of application:
**11.05.2022 Bulletin 2022/19**

(73) Proprietor: **University of Cincinnati**
**Cincinnati, OH 45221-0623 (US)**

(72) Inventors:
• **ZHANG, Xiaowei**
**Cincinnati, OH 45220 (US)**
• **LAZENBY, Robert, A.**
**Cincinnati, OH 45219 (US)**
• **WU, Yao**
**Cincinnati, OH 45221 (US)**
• **WHITE, Ryan**
**Cincinnati, OH 45230 (US)**

(74) Representative: **Plasseraud IP**
**104 Rue de Richelieu**
**CS92104**
**75080 Paris Cedex 02 (FR)**

(56) References cited:
**WO-A1-2018/067521      WO-A2-2018/058028
US-A1- 2007 154 909      US-A1- 2019 178 807
US-A1- 2019 178 807      US-B1- 8 557 050
US-B1- 8 557 050         US-B2- 6 734 305**

• **ZHAI QINGFENG ET AL: "Electrochromic
sensing platform based on steric hindrance
effects for CEA detection", ANALYST, ROYAL
SOCIETY OF CHEMISTRY, UK, vol. 141, no. 13, 26
April 2016 (2016-04-26), pages 3985 - 3988,
XP093063550, ISSN: 0003-2654, DOI: 10.1039/
C6AN00675B**

EP 3 994 446 B1

- **ZHANG XIAOWEI ET AL: "Electrochromic, Closed-Bipolar Electrodes Employing Aptamer-Based Recognition for Direct Colorimetric Sensing Visualization", ANALYTICAL CHEMISTRY, ACS, US, vol. 91, no. 17, 8 August 2019 (2019-08-08), pages 11467 - 11473, XP093063548, ISSN: 0003-2700, DOI: 10.1021/ acs.analchem.9b03013**
- **SANTOS-CANCEL MIRELIS, SIMPSON LAURA W., LEACH JENNIE B., WHITE RYAN J.: "Direct, Real-Time Detection of Adenosine Triphosphate Release from Astrocytes in Three-Dimensional Culture Using an Integrated Electrochemical Aptamer-Based Sensor", ACS CHEMICAL NEUROSCIENCE, vol. 10, no. Iss. 4, 12 February 2019 (2019-02-12), pages 2070 - 2079, XP055780435, DOI: 10.1021/ acschemneuro.9b00033**

(52) Cooperative Patent Classification (CPC): (Cont.)

C-Sets
**C12Q 1/6825, C12Q 2525/205**

## Description

### CROSS REFERENCE TO RELATED APPLICATIONS

This application claims priority to U.S. Provisional Application Serial No. 62/870,392, filed July 3, 2019.

### TECHNICAL FIELD

The present invention relates to the use of aptamers as recognition elements in the development of electrochemical sensors.

### BACKGROUND OF THE INVENTION

Aptamers are nucleic acid or peptide molecules that bind to a target molecule with high specificity. After selection and enrichment, aptamers possess similar affinities to antibody-antigen pairs, but have the advantage of being able to be synthesized using standard methods. As synthetic molecules, aptamers also have unique advantages in the control of their size and their amenability for chemical modification, and as such have been widely developed and applied in the development of sensors. Electrochemical, aptamer-based (E-AB) sensors have emerged in recent years as a platform to detect proteins, small molecules, and inorganic ions, relying on the induced conformational change of oligonucleotide aptamers in the presence of specific analyte. When a target molecule binds to an aptamer, which is tethered to the electrode surface, changes in the aptamer structure are measured by changes in the electrochemical signal of an attached redox label on the aptamer. E-AB sensor response is typically interrogated using a voltammetric technique such as cyclic voltammetry (CV), alternating current voltammetry (ACV), square wave voltammetry (SWV) or chronoamperometry. However, these techniques require additional equipment and are typically performed in a lab. E-AB sensors would be more useful if they had a simple indicator to show a user that a target material has been detected without the need for additional lab equipment.

### SUMMARY OF THE INVENTION

The present invention meets that need by providing a simple and reversible colorimetric sensor that is easy to use and produce. One embodiment of the present invention is a sensor comprising a bipolar electrode combined with a redox indicator aptamer-based sensor and an electrocatalyst, as defined in claim 1. The electrochemical aptamer-based (E-AB) sensor comprises a closed bipolar electrode having a first end and a second end, wherein the first end comprises an electrochromic material and the second end comprises an electrocatalyst and an oligonucleotide aptamer tethered to the second end; wherein the oligonucleotide aptamer is labelled with a redox indicator. The electrochromic material is prussian white.

The redox indicator is methylene blue, the electrocatalyst is ferricyanide. In an embodiment, the electrocatalyst is potassium ferricyanide. In another embodiment, the second end of the bipolar electrode is cathodic.

In another embodiment, a method of detecting an analyte according to claim 4 is provided. The method comprises applying a solution containing the analyte to an electrochemical aptamer-based sensor comprising a closed bipolar electrode and detecting a color change; wherein the aptamer-based sensor is as defined in claim 1.

In one embodiment, the method also involves applying a solution wash to the bipolar electrode after the bipolar electrode has been exposed to the analyte-containing solution. In another embodiment, the solution wash comprises sodium dodecyl sulfate.

### BRIEF DESCRIPTION OF THE DRAWINGS

The foregoing summary, as well as the following detailed description of preferred embodiments of the application, will be better understood when read in conjunction with the appended drawings.

FIG 1A is a depiction of a closed-bipolar electrode, electrochemical, aptamer-based (C-BPE EAB) sensor. FIG 1B is a depiction of the electrode showing that target binding causes a rapid change in color of the indicator electrode; whereas in the absence of a target molecule the color change is slow.

FIG 2 is a graph showing the rate of color change in the indicator electrode is a function of both ATP target concentration and concentration of the redox mediator $Fe(CN)_6^{3-}$.

FIG 3 is a graph showing how the presence of methylene blue (MB) is important for the observed color change in the indicator electrode.

FIGs 4A, 4B and 4C are graphs showing how the rate of color change of the indicator electrode is quantitatively related to the concentration of ATP in the test solution.

FIG 5 is a graph showing how the average value of three determinations based on the discoloring time of 0 mM, 0.1 mM, and 1 mM tobramycin based E-AB sensors.

FIG 6 is a schematic of a current-time trace for the reduction of PB to PW.

FIG 7 is a schematic of current-time traces for the oxidation of PW to PB at various driving voltages.

FIG 8 is a schematic showing how the buffer solution composition affects rate of color change for C-BPE E-AB sensors.

FIG 9 is a table showing the effect of $K_3[Fe(CN)_6]$ and Target.

FIGs 10A and 10B show the results of color changes with different ATP solutions for C-BPE E-AB sensors. FIG 10A is a graphic depiction of 8 mM ATP % color change with EAB sensors prepared using various aptamer solution concentrations (0.2 $\mu$M, 1.0 $\mu$M, 2.0 $\mu$M and 5.4 $\mu$M). FIG 10B is a graphic depiction of 8 mM ATP % color change with EAB sensors prepared using various aptamer solution concentrations (0.2 $\mu$M, 1.0 $\mu$M, 2.0 $\mu$M and 5.4 $\mu$M) at the time of 360 s.

## DETAILED DESCRIPTION OF THE INVENTION

The details of one or more embodiments of the disclosed subject matter are set forth in this document. Modifications to embodiments described in this document, and other embodiments, will be evident to those of ordinary skill in the art after a study of the information provided herein.

The present disclosure may be understood more readily by reference to the following detailed description of the embodiments taken in connection with the accompanying drawing figures, which form a part of this disclosure. It is to be understood that this application is not limited to the specific devices, methods, conditions or parameters described and/or shown herein, and that the terminology used herein is for the purpose of describing particular embodiments by way of example only and is not intended to be limiting. Also, in some embodiments, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

While the following terms are believed to be well understood by one of ordinary skill in the art, definitions are set forth to facilitate explanation of the disclosed subject matter. Unless defined otherwise, all technical and scientific terms used herein have the same meaning as commonly understood by one of ordinary skill in the art to which the disclosed subject matter belongs.

As used herein, the term "about," when referring to a value or to an amount of mass, weight, time, volume, pH, size, concentration or percentage is meant to encompass variations of in some embodiments ±20%, in some embodiments ±10%, in some embodiments ±5%, in some embodiments ±1%, in some embodiments ±0.5%, and in some embodiments ±0.1% from the specified amount, as such variations are appropriate to perform the disclosed method.

As used herein, the term "analyte" means an oligonucleotide or polynucleotide having a sequence to which a particular electrode-bound oligonucleoride is designed to hybridize. It can also refer to a small molecule of the like to which a particular aptamer is designed to hybridize.

As used herein, the term "aptamer" means any polynucleotide molecule (for example, DNA or RNA molecule containing natural or synthetic nucleotides) that has the ability to bind other molecules. For example, aptamers have been selected which bind nucleic acids, proteins, small organic components and even entire organisms.

The particular use of terms "oligonucleotide" and "polynucleotide" should in no way be considered limiting. "Oligonucleotide" is used when the relevant nucleic acid molecules typically comprise less than about 100 bases. "Polynucleotide" is used when the relevant nucleic acid molecules typically comprise more than about 100 bases. Both terms are used to denote DNA, RNA, modified or synthetic DNA or RNA (including but not limited to nucleic acids comprising synthetic and naturally-occurring base analogs, dideoxy or other sugars, and thiols), and PNA or other nucleobase containing polymers. However, probes and/or targets may comprise fewer than or more than 100 bases (inclusive). Accordingly, the terms "oligonucleotide" and "polynucleotide" are used to describe particular embodiments of the invention. The terms in no way define or limit the length of the nucleic acids that may be used to practice the invention.

As used herein, the term "labelled" refers to another molecule being linked, whether covalent or otherwise intercalated to an aptamer.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

In one embodiment, the present invention incorporates a redox indicator such as methylene blue (MB) in an electrochemical sensor. Further, the present invention uses an electrocatalyst, such as a solution-based redox molecule, to electrocatalytically interact with the redox indicator. In one embodiment, the reduced form of MB, leucomethylene blue (LB), is combined with potassium ferricyanide ($[Fe(CN)_6]^{3-}$), as shown in equation 1 and equation 2. The catalytic cycle starts with the rapid, two-electron reduction of MB to LB, which occurs when the electrode is held at a suitably negative potential (Equation 1):

$$MB + 2e^- + H^+ \rightarrow LB \qquad\qquad (Equation\ 1)$$

Two equivalents of $[Fe(CN)_6]^{3-}$ then rapidly oxidize LB back to MB (Equation 2):

$$LB + 2[Fe(CN)_6]^{3-} \rightarrow MB + 2[Fe(CN)_6]^{4-} + H^+ \qquad (Equation\ 2)$$

In the present invention, we take advantage of the catalytic cycle described above by using a redox indicator-tethered aptamer-based sensor with an electrocatalyst and combine it with a closed bipolar electrode (BPE) setup. BPEs are conductors that are polarized in an electric field within an electrolyte solution, such that one end of the electrode becomes cathodic while the opposite end becomes anodic. Such devices have applications in motion control and monitoring, biosensing, the screening of electrocatalysts, analyte separation and enrichment and electrochemical synthesis. BPEs can be classified as either a conventional open-bipolar electrode (O-BPE) system or a closed-bipolar electrode (C-BPE) system. A C-BPE system is used with the E-AB sensor platform of the present invention, since one end of the BPE needs to be aptamer-functionalized while the opposite and separate end is in a reduced state *(vide infra)*.

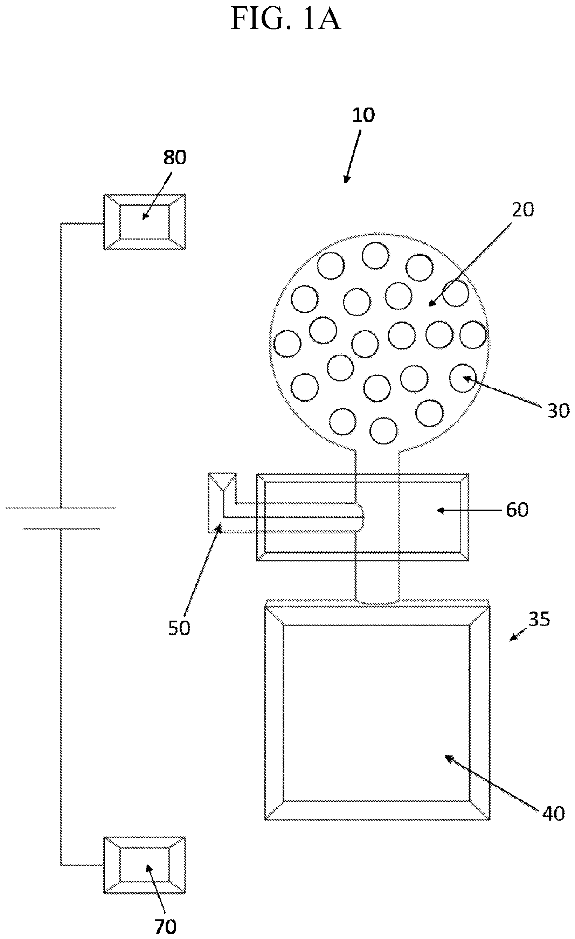

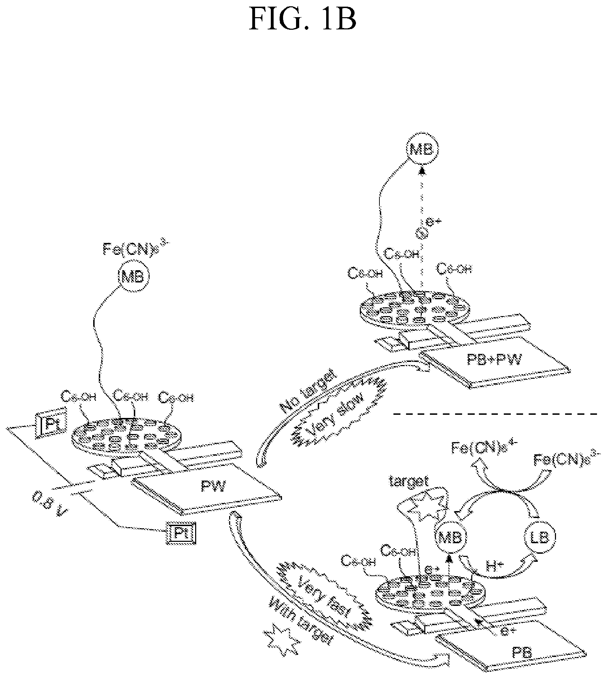

The present invention involves color-changing BPE E-AB sensors, using a C-BPE system. In one embodiment, the BPEs of the present invention may be fabricated using a lithographic process with an indium tin oxide (ITO)-coated glass slide (FIG 1). On one pole, the indicator electrode 35 is decorated with an electrochromic material 40, such as Prussian white (PW), and the other electrode 20 is decorated with gold nanoparticles 30 onto which aptamers are tethered, to form the E-AB sensing monolayer (FIG 1). PW is oxidized to produce Prussian blue (PB) (equation 3). This color change (white to blue) is the signal that can be used for colorimetric analysis using BPEs and E-AB sensors:

$$PW \rightarrow PB + e^- \qquad\qquad (Equation\ 3)$$

Using the electrocatalytic mechanism described above, these three reactions (1, 2 and 3) will support each other. The performance of the E-AB sensors can be visualized directly by the electrochromic reaction. One advantage of a colorimetric sensor is that it can be readily combined with smartphone algorithms for analysis.

For a visual example of the implementation of these mechanisms, we refer to FIG 1A. This figure is a depiction of one embodiment of the present invention. It shows a closed-bipolar electrode, electrochemical, aptamer-based (C-BPE EAB) sensor **10** that couples specific target binding of an analyte on a sensing electrode **20** with a color change of an indicator electrode **35** coated with an electrochromic material **40.** In one embodiment, the electrochromic material is Prussian white (PW). In one embodiment, the sensing electrode **20** is decorated with gold nanoparticles **30,** which provide tether points for aptamer. The sensor **10** further incorporates an indicator electrode preparation contact **50** and an electrode separation material **60** to separate the sensing electrode **20** and the indicator electrode **35.** In one embodiment, the electrode separation material **60** is a PDMS membrane. A quasi-reference counter electrode **70** and a working electrode **80** are held at a driving potential to polarize the sensor 10. In one embodiment, these electrodes are Pt sheet electrodes. In one embodiment, the driving potential is about -0.8 V.

FIG 1B. is a depiction of the electrode showing that target binding increases the rate of MB reduction (reaction 1) and thus $Fe(CN)_6^{3-}$ reduction (reaction 2), which in turn increases the rate of PW oxidation to PB (reaction 3) causing a rapid change in color of the indicator electrode. (Right, top) Conversely, in the absence of target molecule, the color change of PW to PB is slow. The color change in the indicator electrode serves as the optical transduction of the sensor.

The present invention is a simple and reversible colorimetric sensor that combines the concentration-dependent aptamer-target binding phenomenon with a universal C-BPE, in which $K_3[Fe(CN)_6]$ is used as a catalyst. Compared with the traditional E-AB sensor platform, the C-BPE architecture represents a quantitative colorimetric sensor, which is relatively simple and easy to use and produce. Using this method, sensors for ATP and tobramycin have been fabricated and used to accurately determine target molecule concentration. This phenomenon indicates that an E-AB sensor based on a C-BPE is very useful for easy-to-interpret POC devices that do not require a potentiostat for use. With further characterization and optimization of the operational and fabrication parameters, the color changing C-BPE E-AB sensor strategy of the present invention opens up a new class of color-changing, point-of-use sensors.

Redox Indicator

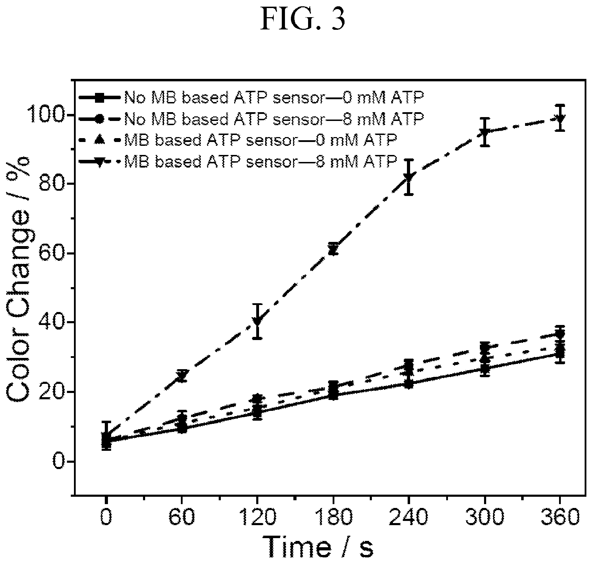

FIG 3 shows how the presence of a redox indicator, such as methylene blue (MB), is important for the observed color change in the indicator electrode. More specifically, when sensors are fabricated with unlabeled aptamer (unlabeled ATP aptamer), no appreciable change in rate of color change or percent color change are observed. Conversely, when the aptamer is labelled with MB, the rate of color change and percent color change are significantly larger. Error bars represent the standard deviation of different measurements on a single sensor device (n = 3), in which the sensor was washed (10% SDS) and reset (PB to PW) between each run.

The redox indicator of the present invention is the reducible/oxidizable chemical moiety

Electrocatalyst

The electrocatalyst of the present invention is ferricyanide.

Electrochromic Material

The electrochromic material of the present invention is prussian white.

Aptamer

In one embodiment, the aptamer of the present invention is an aptamer that binds to aminoglycoside antibiotics like tobramycin. In another embodiment, the aptamer can be sequences that bind to small molecules like cocaine, adenosine triphosphate, methamphetamine, polychlorinated biphenyls, kanamycin, peptides and proteins like $17\beta$-estradiol, progesterone, neuropeptide Y, cells both mammalian and bacterial, and viral particles.

**EXAMPLES**

C-BPEs that incorporate an aptamer-based sensor on an Au nanoparticle-decorated substrate were fabricated and tested under a range of conditions. To obtain a colorimetric sensor with optimal performance, parameters such as applied potential, buffer composition, and aptamer concentration were varied. The sensors were then tested using a range of target molecule concentrations, using ATP and tobramycin as model systems.

Chemicals and Materials

Ethanol, hydrochloric acid (HCl), sodium chloride (NaCl), potassium chloride (KCl), magnesium chloride ($MgCl_2$), ferric chloride ($FeCl_3$), sodium bicarbonate ($NaHCO_3$), potassium ferricyanide ($K_3[Fe(CN)_6]$), hydrochloric acid (HCl), 4-(2-hydroxyethyl)-1-piperazineethanesulfonic acid (HEPES), 6-mercapto-1-hexanol ($C_6$-OH), adenosine triphosphate (ATP), tobramycin, chlorauric acid, tris-(2-carboxyethyl) phosphine hydrochloride (TCEP), 2-amino-2-(hydroxymethyl)-1,3-propanediol (Tris base), sulfuric acid ($H_2SO_4$), 10% sodium dodecyl sulfate (SDS), polydimethylsiloxane (PDMS) and calcium chloride ($CaCl_2$) were used as received (Sigma-Aldrich, St. Louis, MO). The interrogation buffer was a Tris buffer (50 mM Tris base, 10 mM KCl, 100 mM NaCl, 50 mM $MgCl_2$, pH = 7.4). Aptamers were selected that bind specifically to ATP and tobramycin, with a thiol group on the 5' end and with or without a MB label on the 3' end. Three DNA aptamer sequences were used: an ATP-specific aptamer with MB-label (5'-HSC$_6$-CTGGGGGAGTATTGCGGAGGAAA-MB-3'), the same ATP-specific aptamer without the MB-label (5'-HSC$_6$-CTGGGGGAGTATTGCGGAGGAAA-3') and a tobramycin-specific aptamer (5'-HSC$_6$-GGGACTTGGTTTAGGTAATGAGTCCC-MB-3'), all purchased from Biosearch Technologies, Inc. (Novato, CA) and used as received. ITO-coated glass substrates (resistance: ~6 $\Omega$/square) were obtained from CSG Holding Co., Ltd. (Shenzhen, China). A photosensitive dry photoresist sheet (MonkeyJack 30 cm $\times$ 1 m), and a transparency film for laser printers (Trulam, 4 mil thickness) were purchased from Amazon. All other chemicals were used as received (analytical reagent grade), without any further purification. All solutions were made with deionized (DI) water, purified through a water purification system (18.2 M$\Omega$ cm, Milli-Q Advantage A10, Millipore, Billerica, MA).

Fabrication of Patterned ITO Electrodes

The photosensitive dry film was employed to photolithographically fabricate patterned ITO electrodes. The photosensitive dry film was applied directly onto the clean ITO substrate. A photomask with the designed patterned template was fabricated by printing the BPE patterns using a laser printer (MFC-9330CDW, Brother), and was positioned on the coated ITO substrate. This was exposed to direct sunlight for 30 seconds, which hardened the film for the desired pattern (dry film is negative). The sample was submerged in 0.1 M NaOH to remove the exposed photoresist layer. The exposed ITO was etched by the $FeCl_3$-HCl solution and the patterned ITO electrodes were obtained by removing the remaining photoresist with 1 M NaOH. Finally, the C-BPE system was achieved by direct bonding of a PDMS membrane (by physical contact, no adhesive) to create the two reservoirs on the patterned ITO substrate, thus separating the opposing ends of the BPE.

Deposition of Au and PB on the ITO and E-AB Sensor Fabrication

Au was electrodeposited on the cathodic end of the ITO bipolar electrode (3 mm diameter circle geometry, FIG 1A), using a solution containing 0.01 M $HAuCl_4$ and applying a potential of -0.6 V directly to a contact positioned in the center of the bipolar electrode (see FIG 1A) for 60 s. This resulted in a surface with a roughness factor of $1.53 \pm 0.33$, based on a comparison of the geometric area and electrochemically determined surface area, obtained by measuring the charge associated with the reduction of the gold oxide layer in cyclic voltammetry (in 0.05 M $H_2SO_4$) and using the ratio 422 $\mu$C cm$^{-2}$. Prussian blue (PB) was made by combining 2.5 mM $FeCl_3$ and 2.5 mM $K_3[Fe(CN)_6]$, which was deposited on the bipolar anode (3 mm length square geometry, FIG 1A) of the C-BPE, by applying a potential of 2.2 V for 300 s. Prior to sensor use, PB was reduced to PW in an initialization step, by applying a potential of 1.2 V for 18 s (in 0.1 M NaCl and 0.1 M HCl). The same solution was used at this pole for sensor interrogation.

The Au acted as a substrate to which aptamer could be tethered. 1 $\mu$L aptamer solution (200 $\mu$M) and 1 $\mu$L TCEP (10 mM) were added together, and allowed to stand for 1 hour (to reduce any 5' disulfide bonds). The solution was diluted to 2 $\mu$M using 98 $\mu$L of buffer (20 mM Tris and 100 mM NaCl, pH = 7.4). The 100 $\mu$L solution was placed on the Au nanoparticle-coated electrode of ITO for 1 hour to allow for self-assembled monolayer formation, in a covered petri dish to minimize evaporation. The surface was rinsed with DI water, followed by incubation in 2 mM $C_6$-OH for 12 hours, to remove non-specifically adsorbed DNA and passivate the surface, in a covered petri dish.[35] Probe aptamer surface density was determined by integrating the MB reduction peak in CV, at 20, 50 and 100 mV s$^{-1}$ scan rates.

Electrochemical Measurements and Sensor Interrogation

The electrochemical measurements were performed at room temperature using a CHI 660D (CH Instruments, Austin, TX) Electrochemical Workstation. Chronoamperometry was used for investigating optimal holding potentials for the PB to PW sensor initialization and the PW to PB sensor interrogation. The C-BPE was positioned between two Pt sheet electrodes (Surepure Chemetals, L.L.C., 2.5 cm $\times$ 6.5 cm, 50 $\mu$m thickness), which were held at a potential to polarize the C-BPE sensor. The Pt electrode at the Au-deposited end was the working electrode and the electrode at the electrochromic end was the quasi-reference counter electrode (QRCE). Unless otherwise stated, all potentials refer to the potential applied at the Pt working electrode.

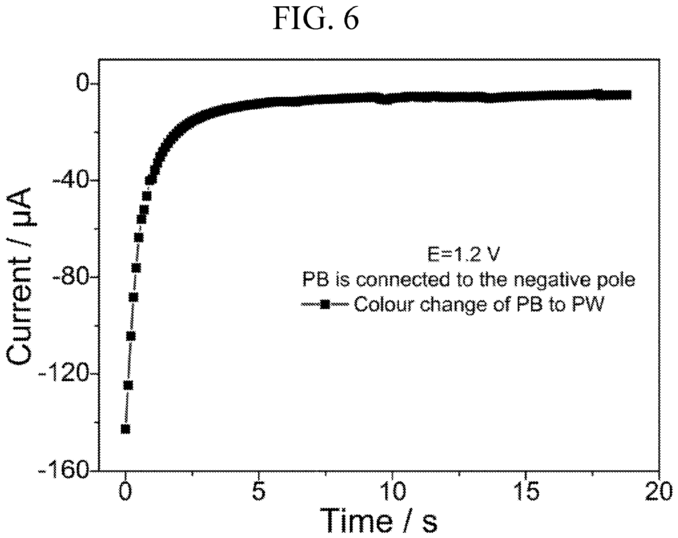

Prior to testing the C-BPE E-AB sensor device, the PB-coated anode was reduced to PW, so that it could be oxidized back to PB anodically. To ensure an even distribution, a potential of 1.2 V was applied directly to the center contact of the bipolar electrode (not the Pt working electrode) to reduce PB to PW, for at least 10 s (FIG 6). Any testing of the sensor should immediately follow this sensor reset (PB to PW), so that the determined color change starts close to 0% (i.e. fully PW). To account for sensor variability, the same sensor was tested under multiple conditions, e.g. a range of concentrations. Between each experiment, the sensor was placed in 10% SDS for 15 min and rinsed with DI water for 20 s, prior to sensor reset.

Images of the C-BPEs were taken using a smartphone (iPhone 7 Plus, 12 MP camera) and semi-quantitative analysis was performed in Adobe Photoshop. Analysis involved selecting an area from the image of the PW/PB anodic end of the device (free from artifacts) and tracking the blue color intensity of these pixels over time. Color changes were determined as a percentage using:

$$\% \text{ Color change} = (\eta / \eta_{\text{final}}) \times 100\% \qquad \text{(Equation 4)}$$

where $\eta$ is the blue color intensity, and $\eta_{\text{final}}$ is the final color of PB (fully oxidized), taken from a single reference device that had been fully converted from PW to PB. Note that a light screening box was not used, and some images contain light reflections in the droplet and artifact shadows, but the camera was in a fixed position, so the same area of the image could be monitored and compared for the effective color change over time in this proof of principle setup.

Table 1 shows buffer composition with % color change with and without 16 mM ATP.

Table 1

| Buffer No. | [Tris] / mM | [NaCl] / mM | MgCl$_2$ / mM | [K$_3$[Fe(CN)$_6$]] / μM | % color change without ATP (after time) | % color change with 16 mM ATP (after time) |
|---|---|---|---|---|---|---|
| 1 | 1 | 5 | 0.025 | 200 | 28 ± 1 (6 mins) | 54 ± 2 (6 mins) |
| 2 | 2 | 10 | 5 | 200 | 41 ± 2 (6 mins) | 100 ± 6 (6 mins) |
| 3 | 10 | 50 | 2.5 | 200 | 83 ± 3 (6 mins) | 100 ± 5 (2 mins 38 s) |
| 4 | 20 | 100 | 5 | 200 | 100 ± 7 (2 mins 51 s) | 100 ± 3 (2 min 3 s) |

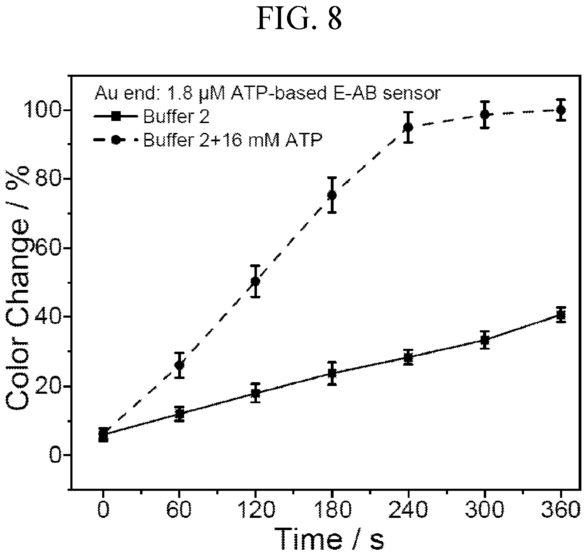

FIG 8 shows the determined color changes of 0 mM and 16 mM ATP-specific BPE E-AB sensors (n = 3) in Buffer 2 (2 mM Tris, 10 mM NaCl, 5 mM MgCl$_2$ and 200 μM K$_3$[Fe(CN)$_6$]). Error bars represent the standard deviation of different measurements on a single device (n = 3).

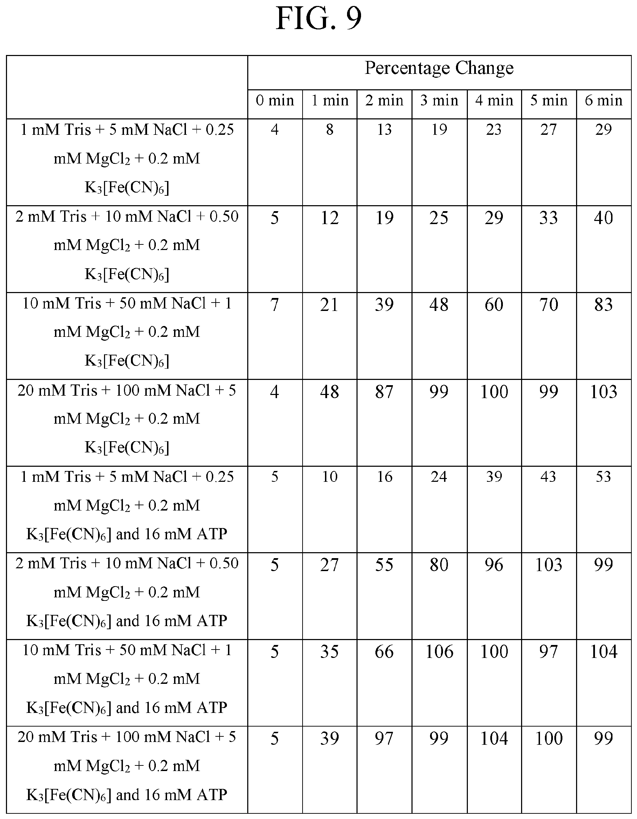

FIG 9 shows the color change with different buffer solutions: I. (1) 1 mM Tris, 5 mM NaCl, 25 μM MgCl$_2$ and 200 μM K$_3$[Fe(CN)$_6$]; (2) 2 mM Tris, 10 mM NaCl, 5 mM MgCl$_2$ and 200 μM K$_3$[Fe(CN)$_6$]; (3) 10 mM Tris, 50 mM NaCl, 2.5 mM MgCl$_2$ and 200 μM K$_3$[Fe(CN)$_6$]; (4) 20 mM Tris, 100 mM NaCl, 5 mM MgCl$_2$ and 200 μM K$_3$[Fe(CN)$_6$]. II. As for I, with 16 mM ATP. Images were recorded at 0 min (left) and at 1 min intervals thereafter.

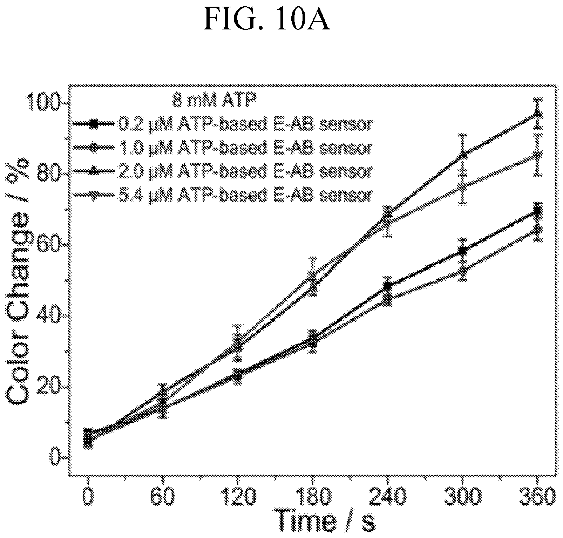

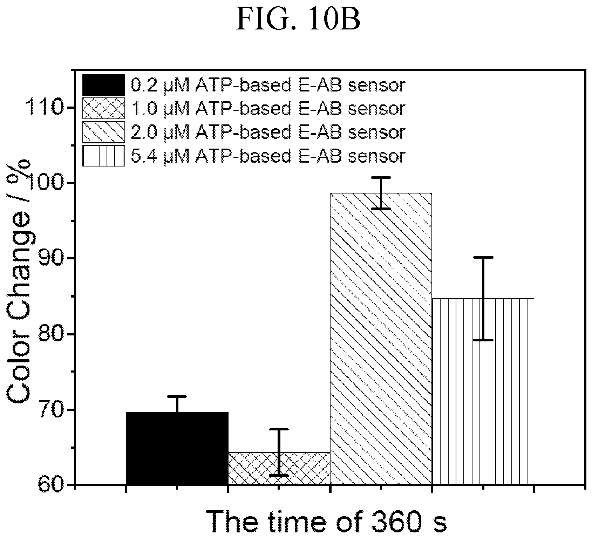

As is common in the performance of E-AB sensors, the surface coverage of the aptamer probe on the electrode surfaces affect signaling of the resulting sensor. We employed concentrations of aptamer ranging from 0.2 μM, 1 μM, 2.0 μM and 5.4 μM, leading to a range of packing densities of aptamer on the electrode surface. Each was tested in the presence and absence of target analyte (0, 8 and 16 mM ATP). At the high target concentration (16 mM ATP) the rate of color change was rapid, such that the end point (100%) was reached in less than 3 mins for sensor surfaces prepared with 2.0 μM and 5.4 μM aptamer. The lower target concentration (8 mM ATP) showed a slower response. Sensor surfaces prepared with 2 μM aptamer during modification (for ATP-specific E-AB sensors) were determined to be optimal as surfaces prepared with 0.2 μM and 1 μM aptamer were slow to reach maximum color change, reaching just 69.7% and 64.3% after 6 minutes, respectively. The concentration of 2 μM ATP-based E-AB sensors reached the endpoint (100%) in 6 minutes with 8 mM ATP. Moreover, after 6 minutes the 5.4 μM ATP-based E-AB sensor reached a value of 84.3% color change, i.e. not faster than the sensor prepared using 2 μM ATP aptamer. FIG 10A is a graphic depiction of 8 mM ATP % color change with EAB sensors prepared using various aptamer solution concentrations (0.2 μM, 1.0 μM, 2.0 μM and 5.4 μM). FIG 10B is a graphic depiction of 8 mM ATP % color change with EAB sensors prepared using various aptamer solution concentrations (0.2 μM, 1.0 μM, 2.0 μM and 5.4 μM) at the time of 360 s.

Example 1 - Electrode Potential Optimization for PW Conversion to PB

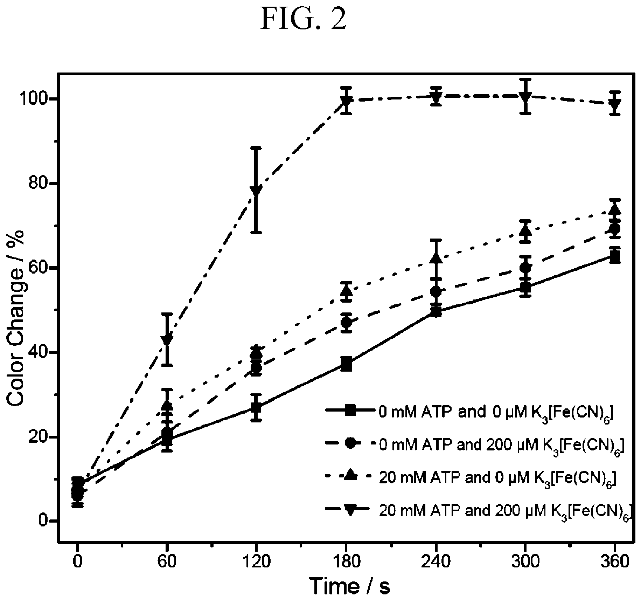

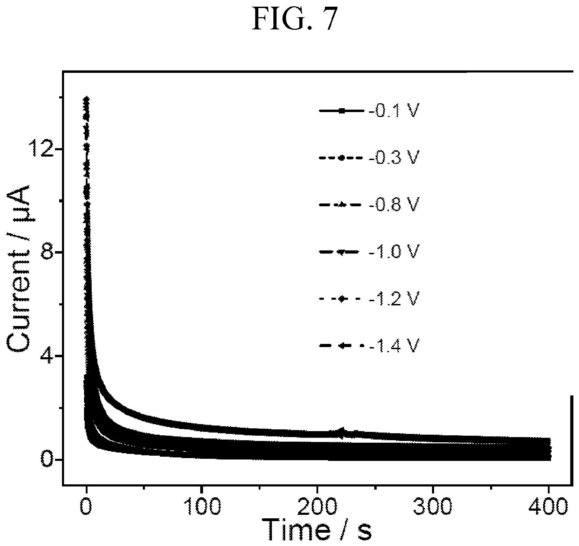

In order to optimize the performance of the C-BPE based sensor, several parameters that affect the rate of color change, and thus signal, of the indicator electrode were explored. The first parameter tested was the driving potential employed during sensing. A range of driving potentials (-0.1, -0.3, -0.8, -1.0, -1.2 and -1.4 V) were applied for 400 s (FIG 7), to find the optimal speed of color change. The sensor device should operate quickly for point-of-care (POC) applications, but respond slowly enough that changes in rate of color change can be properly observed. For highly negative potentials <-1.0 V, the color change from PW oxidation to PB was rapid (47 s or less). When small negative potentials are applied (-0.1 through -0.3 V), the color change reached 18% ± 6% and 21% ± 8% in 6 mins, respectively. These magnitudes of the observed color change are subtle, as the applied driving potential was not high enough to oxidize PW to PB. 6 mins was discretionarily chosen as the time for comparison of different sensors, as it was sufficiently long enough to show a clear difference in response between tests with and without target. At more negative potentials, it was found that the speed at which PW changes to PB increased. Using an applied potential of -0.8 V, for example, resulted in a color change of 35% ± 7% within 6 mins, i.e., significantly more than air oxidation alone. When even more negative potentials were applied, such as -1.0, -1.2 and -1.4 V, color change reached 100% in less than 6 mins. For example, at -1.0 V, maximum signal change (99% ± 6%) was observed after 47 s. -0.8 V was selected as the optimal potential for all further experiments, since it was sufficiently negative to oxidize PW (faster than air oxidation), but not so negative that the speed of color change would diminish the sensor's sensitive performance. Of note, some color change is observed as a result of PW oxidation in air. This explains the presence of some PB at t = 0 s in FIG 2, due to a delay in sensor interrogation after the initialization potential was applied (for PB to PW conversion).

Example 2 - Addition of K$_3$[Fe(CN)$_6$] and Target Increases Rate of Color Change

Adding ferricyanide to the solution in combination with target analyte at the aptamer-based sensing electrode results in a higher rate of color change at the indicator electrode. To demonstrate this, a series of experiments were performed comprising tests with C-BPEs with or without $K_3[Fe(CN)_6]$ and with or without target (ATP), which were used to show the electrocatalytic redox cycling effect (FIG 2). When both $K_3[Fe(CN)_6]$ (200 $\mu$M) and ATP (20 mM) were present, the color change reached completion (100%) after ~2 mins (2 mins 18 s). If either ATP or $K_3[Fe(CN)_6]$ or both were absent from the solution, the color change was only about 29% - 42% at this same time (FIG 2). The color change is most obvious when both $K_3[Fe(CN)_6]$ and ATP are present together and as such, this serves as the basis of the sensing mechanism.

FIG 2 is a graph showing the rate of color change in the indicator electrode is a function of both ATP target concentration and concentration of the electrocatalyst (for example, $Fe(CN)_6^{3-}$). (Left) Indicator electrode color change is observable with the naked eye after 120 s when both ATP and $Fe(CN)_6^{3-}$ are present in the sample solution. (Right) Quantitatively, the rate at which the color change occurs is 0.60% $s^{-1}$ when both ATP and $Fe(CN)_6^{3-}$ are present compared to 0.17% $s^{-1}$ for the other conditions tested. More specifically, when both target and mediator are present, a 100% signal color change is observed after 180 s compared to ~20% using the other conditions tested after the same interval. Error bars represent the standard deviation of three measurements using the same device (n = 3). 100% signal change was defined by the blue color of a reference chip that had fully converted PW to PB.

Example 3 - MB Label Increases Rate of Color Change in Presence of Target for E-AB Sensor

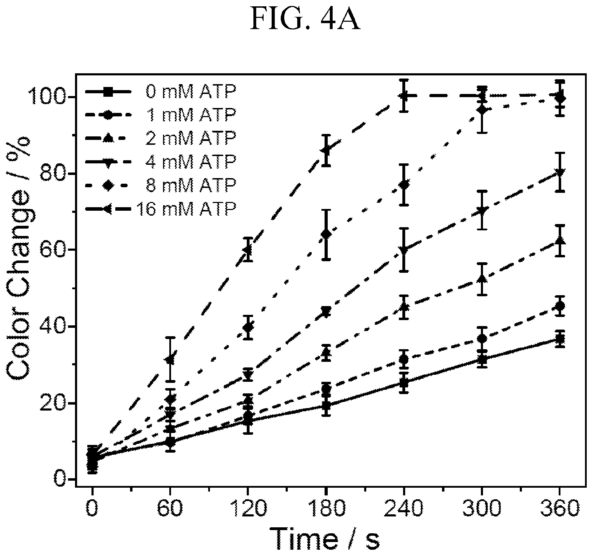

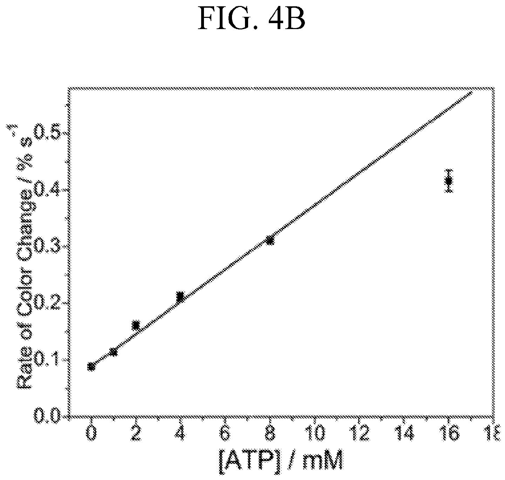

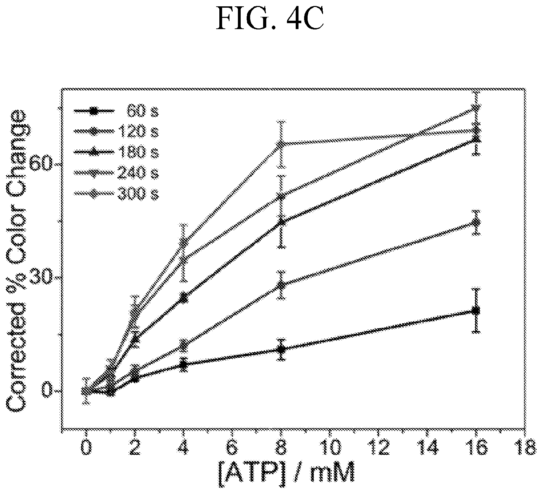

To demonstrate the effect of the MB redox label on the aptamer probes, tests were conducted using sensors fabricated with the ATP-specific aptamer sequence without an appended redox label (FIG 1, FIG 3 and FIG 9). The unlabeled aptamer probe yielded no observable difference in the rate of color change with 0 mM and 8 mM ATP, changing 31% $\pm$ 3% and 37% $\pm$ 2% after 6 mins, respectively. This result indicates that both reactions (1) and (2) do not proceed without MB. Conversely, when the aptamer probe possesses the MB label, color changes of 33% $\pm$ 5% and 99% $\pm$ 4% are observed after 6 mins, for 0 mM and 8 mM ATP, respectively (FIG 4). This result further demonstrates the catalytic effect of $K_3[Fe(CN)_6]$.

Example 4 - Target Concentration Dependence of C-BPE E-AB Sensors Specific to ATP and Tobramycin.

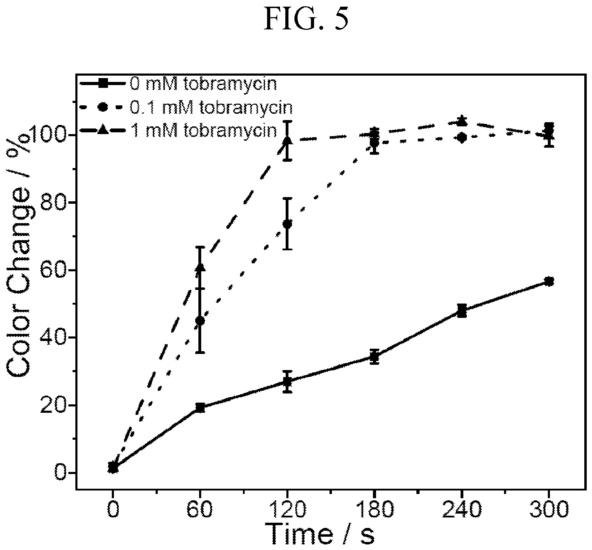

The magnitude and rate of color change of the C-BPE E-AB sensor is quantitatively related to concentration of target analyte and the trend is general for different aptamer-target binding partners. For example, sensors fabricated against ATP (FIGs 4A-C) and tobramycin (FIG 5) both demonstrate target-concentration, color change dependence. With an ATP-specific E-AB sensor, the color change with a range of concentrations of ATP (0, 1, 2, 4, 8 and 16 mM) exhibited a linear relationship (FIGs 4A-C). Meanwhile, FIG 4A shows the color change from white to blue occurred at different rates. A calibration of the sensor was constructed, which was linear in this high concentration for the rate of color change, which was linear in this high concentration range (FIG 4B). It should be noted that each ATP-specific E-AB sensor will potentially require individual calibration as the nature of the electrode surfaces can vary and thus vary the rate of signal change (more discussion below). An alternative calibration strategy is to measure the color change after a specific time, chosen for optimal sensitivity and dynamic range (FIG 4C).

C-BPE E-AB sensors were also made for tobramycin and tested against different concentrations of this target molecule (FIG 5). Similar to the response of the C-BPE E-AB sensor for ATP, the tobramycin sensor also exhibited a concentration dependent rate of color change, showing this electrochromic sensor is general for multiple E-AB sensors. We have demonstrated that E-AB sensors can be used with the C-BPE system, only if ferricyanide is added to catalyze the reaction of LB to MB, which results in an increased rate of conversion of PW to PB in the presence of target.

Using a simple DC power supply to polarize the C-BPE, and a smartphone camera to monitor the rate of color change of the sensor, this C-BPE E-AB colorimetric sensor can be used for the rapid detection of specific analytes in POC applications, without the need for a potentiostat and software for interpreting voltammetric data. The use of smartphones in POC colorimetric sensors has been realized for pH, protein content and glucose sensing. Moreover, this type of sensor is cheap to produce and relatively simple to make on a large scale. Furthermore, individual sensors are reusable for multiple times, with a solution wash (10% SDS) and the application of a potential to reset the sensor.

It should be noted here that there are several operational parameters that can be optimized to further improve the sensitivity and reproducibility of the described C-BPE devices. More specifically, the electrochromic film material and thickness, the surface quality of the gold substrate electrode, the size and position of the device with respect to the Pt working and quasi-reference electrodes and the electrocatalyst concentration can all affect sensor-to-sensor variability. PW was selected as the material for the electrochromic film, since the color change is reversible and repeatable. However, under no driving potential PW will convert to PB in ~30 mins (c.f. 6 mins using a driving potential of -0.8 V). If this background process was reduced, or eliminated, a higher sensitivity could be achieved, and a lower limit of detection. This may be accomplished using a different electrochromic material that does not change color under the applied driving potential required for the E-AB sensor to function. The sensing electrode employs aptamers with a surface probe coverage, $\Gamma$, of 3.5 $\pm$ 1.8 $\times 10^{12}$ molecules $cm^{-2}$ (n = 3). Under identical aptamer modification conditions (1 hour incubation in 2 $\mu$M aptamer solution), this coverage is largely governed by the surface roughness and quality of the underlying electrodeposited gold layer, which could be improved to give more reproducible sensor performance. Another consideration for the use of BPEs is the position of the working electrode and QRCE with respect to the BPE. Changes in the positions of these electrodes will result in a difference in induced polarization, which could inhibit sensor performance. Additionally, a higher electrocatalyst concentration may result in faster conversion of LB to MB, thus faster conversion of PW to PB in the presence of target molecule.

Regarding FIG 6, the colorimetric C-BPE device relies on the electrochromic thin film starting in the reduced state, i.e. prussian white (PW). This requires the reduction of prussian blue (PB) to prussian white, to initialize the sensor, and was achieved using an applied potential difference of 1.2 V. FIG 6 shows the current-time trace for the reduction of PB to PW in 0.1 M KCl and 0.1 M HCl, using an applied potential of 1.2 V.

Regarding FIG 7, a range of potential differences were explored to polarize the bipolar electrode. Note that, these are the potentials applied to the Pt working electrode with respect to the Pt quasi-reference counter electrode (QRCE). The actual potential felt at the PB thin film electrode will differ from this. FIG 7 shows the Current-time traces for the oxidation to PW to PB at various driving voltages (-0.1, -0.3, -0.8, -1.0, -1.2 and -1.4 V vs. Pt) on C-BPE E-AB sensors in 20 mM Tris, 100 mM NaCl, 5 mM $MgCl_2$ and 200 $\mu$M $K_3[Fe(CN)_6]$.

A series of four buffer conditions were investigated before and after adding ATP (Table 1, FIG 8). The sensor was interrogated in each buffer prior to adding ATP, then the sensor was placed in 10% SDS for 15 min and rinsed with DI water for 20 s, after which 16 mM ATP was added and the sensor was interrogated again. Buffer 1 showed a 28% $\pm$ 1% color change (white to blue) when no ATP was added, and 54% $\pm$ 2% when 16 mM ATP was added. Buffer 2 showed a 41% $\pm$ 2% color change in the absence if ATP and 100% $\pm$ 6% after 6 mins in the presence of 16 mM ATP. Buffer 3 gave color changes that were too fast, even in the absence of ATP, with 83% $\pm$ 3% after 6 mins without ATP and 100% $\pm$ 5 after 2 mins 38s with 16 mM ATP. The conditions of Buffer 4 show no significant difference between the presence and absence of ATP, reaching 100% after 2 mins 51s and 2 min 3 s, respectively. Buffer 2 was therefore selected as the most appropriate buffer for future experiments, given that the color change with and without ATP was greatest over the experimental time window (4 mins), as seen in FIG 8. A table of the results of this analysis are shown in FIG 9.

It is to be further understood that where descriptions of various embodiments use the term "comprising," and / or "including" those skilled in the art would understand that in some specific instances, an embodiment can be alternatively described using language "consisting essentially of" or "consisting of."

While particular embodiments of the present invention have been illustrated and described, it would be obvious to one skilled in the art that various other changes and modifications can be made within the scope of the appended claims.

## Claims

1. An electrochemical aptamer-based (E-AB) sensor comprising a closed bipolar electrode having a first end and a second end, wherein the first end comprises an electrochromic material and the second end comprises an electrocatalyst and an oligonucleotide aptamer tethered to the second end; wherein the oligonucleotide aptamer is labelled with a redox indicator, wherein:

   - the electrochromic material is prussian white,
   - the redox indicator is methylene blue,
   - the electrocatalyst is ferricyanide.

2. The sensor of claim 1 wherein the electrocatalyst is potassium ferricyanide.

3. The sensor of claim 1 wherein the second end of the bipolar electrode is cathodic.

4. A method of detecting an analyte comprising applying a solution containing the analyte to an electrochemical aptamer-based sensor according to claim 1 and detecting a color change.

5. The method of claim 4 further comprising applying a solution wash to the bipolar electrode after the bipolar electrode has been exposed to the analyte-containing solution.

6. The method of claim 5 wherein the solution wash comprises sodium dodecyl sulfate.

## Patentansprüche

1. Elektrochemischer Sensor auf Aptamerbasis (E-AB), umfassend eine geschlossene bipolare Elektrode, welche ein erstes Ende und ein zweites Ende aufweist, wobei das erste Ende ein elektrochromes Material umfasst und das zweite Ende einen Elektrokatalysator und ein Oligonukleotid-Aptamer umfasst, welches an das zweite Ende gebunden ist; wobei das Oligonukleotid-Aptamer mit einem Redoxindikator markiert ist, wobei:

- das elektrochrome Material Preußischweiß ist,
- der Redoxindikator Methylenblau ist,
- der Elektrokatalysator Ferricyanid ist.

2. Sensor nach Anspruch 1, wobei der Elektrokatalysator Kaliumferricyanid ist.

3. Sensor nach Anspruch 1, wobei das zweite Ende der bipolaren Elektrode kathodisch ist.

4. Verfahren zum Nachweisen eines Analyten, umfassend Aufbringen einer Lösung, welche den Analyten enthält, an einen elektrochemischen Sensor auf Aptamerbasis gemäß Anspruch 1 und Nachweisen einer Farbänderung.

5. Verfahren nach Anspruch 4, ferner umfassend Aufbringen einer Waschlösung auf die bipolare Elektrode, nachdem die bipolare Elektrode der Analyt-haltigen Lösung ausgesetzt worden ist.

6. Verfahren nach Anspruch 5, wobei die Waschlösung Natriumdodecylsulfat umfasst.


**Revendications**

1. Capteur électrochimique à base d'aptamère (E-AB) comprenant une électrode bipolaire fermée ayant une première extrémité et une seconde extrémité, dans lequel la première extrémité comprend un matériau électrochrome et la seconde extrémité comprend un électrocatalyseur et un aptamère oligonucléotidique fixés à la seconde extrémité ; dans lequel l'aptamère oligonucléotidique est marqué avec un indicateur redox, dans lequel :

- le matériau électrochrome est le blanc de Prusse,
- l'indicateur redox est le bleu de méthylène,
- l'électrocatalyseur est un ferricyanure.

2. Capteur selon la revendication 1, dans lequel l'électrocatalyseur est du ferricyanure de potassium.

3. Capteur selon la revendication 1, dans lequel la seconde extrémité de l'électrode bipolaire est cathodique.

4. Procédé de détection d'un analyte comprenant l'application d'une solution contenant l'analyte sur un capteur électrochimique à base d'aptamère selon la revendication 1 et la détection d'un changement de couleur.

5. Procédé selon la revendication 4, comprenant en outre l'application d'une solution de lavage sur l'électrode bipolaire après que l'électrode bipolaire a été exposée à la solution contenant l'analyte.

6. Procédé selon la revendication 5, dans lequel la solution de lavage comprend du dodécylsulfate de sodium.

# FIG. 1A

# FIG. 1B

# FIG. 2

# FIG. 3

FIG. 4A

FIG. 4B

FIG. 4C

# FIG. 5

FIG. 6

FIG. 7

# FIG. 8

# FIG. 9

| | Percentage Change | | | | | | |
|---|---|---|---|---|---|---|---|
| | 0 min | 1 min | 2 min | 3 min | 4 min | 5 min | 6 min |
| 1 mM Tris + 5 mM NaCl + 0.25 mM MgCl$_2$ + 0.2 mM K$_3$[Fe(CN)$_6$] | 4 | 8 | 13 | 19 | 23 | 27 | 29 |
| 2 mM Tris + 10 mM NaCl + 0.50 mM MgCl$_2$ + 0.2 mM K$_3$[Fe(CN)$_6$] | 5 | 12 | 19 | 25 | 29 | 33 | 40 |
| 10 mM Tris + 50 mM NaCl + 1 mM MgCl$_2$ + 0.2 mM K$_3$[Fe(CN)$_6$] | 7 | 21 | 39 | 48 | 60 | 70 | 83 |
| 20 mM Tris + 100 mM NaCl + 5 mM MgCl$_2$ + 0.2 mM K$_3$[Fe(CN)$_6$] | 4 | 48 | 87 | 99 | 100 | 99 | 103 |
| 1 mM Tris + 5 mM NaCl + 0.25 mM MgCl$_2$ + 0.2 mM K$_3$[Fe(CN)$_6$] and 16 mM ATP | 5 | 10 | 16 | 24 | 39 | 43 | 53 |
| 2 mM Tris + 10 mM NaCl + 0.50 mM MgCl$_2$ + 0.2 mM K$_3$[Fe(CN)$_6$] and 16 mM ATP | 5 | 27 | 55 | 80 | 96 | 103 | 99 |
| 10 mM Tris + 50 mM NaCl + 1 mM MgCl$_2$ + 0.2 mM K$_3$[Fe(CN)$_6$] and 16 mM ATP | 5 | 35 | 66 | 106 | 100 | 97 | 104 |
| 20 mM Tris + 100 mM NaCl + 5 mM MgCl$_2$ + 0.2 mM K$_3$[Fe(CN)$_6$] and 16 mM ATP | 5 | 39 | 97 | 99 | 104 | 100 | 99 |

# FIG. 10A

# FIG. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62870392